(19) Europäisches Patentamt
European Patent Office
Office européen des brevets

(11) Veröffentlichungsnummer: **0 052 731**
**B1**

(12) # EUROPÄISCHE PATENTSCHRIFT

(45) Veröffentlichungstag der Patentschrift: **17.04.85**

(21) Anmeldenummer: **81108068.8**

(22) Anmeldetag: **08.10.81**

(51) Int. Cl.⁴: **C 07 D 311/70**, C 07 D 311/72, C 07 D 407/04, C 07 D 311/22 // (C07D407/04, 311:22, 319:06)

(54) **Chromanderivate sowie Verfahren zu deren Herstellung.**

(30) Priorität: **24.11.80 DE 3044109**

(43) Veröffentlichungstag der Anmeldung: **02.06.82 Patentblatt 82/22**

(45) Bekanntmachung des Hinweises auf die Patenterteilung: **17.04.85 Patentblatt 85/16**

(84) Benannte Vertragsstaaten: **BE CH DE FR GB IT LI NL**

(56) Entgegenhaltungen:
**CH - A - 475 977**
**CH - A - 602 700**
**DE - A - 2 611 910**
**DE - B - 2 160 103**
**US - A - 4 100 175**

(73) Patentinhaber: **BASF Aktiengesellschaft, Carl-Bosch-Strasse 38, D-6700 Ludwigshafen (DE)**

(72) Erfinder: **Vogel, Friedrich, Dr., Im kleinen Letten 3, D-6706 Wachenheim (DE)**
Erfinder: **Paust, Joachim, Dr., Ringstrasse 3, D-6701 Neuhofen (DE)**

**EP 0 052 731 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft neue Chromanderivate der allgemeinen Formel I

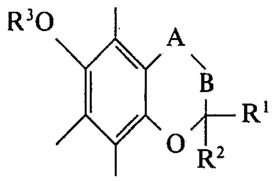

(I)

in welcher die Substituenten folgende Bedeutung haben:

| | | |
|---|---|---|
| A — B | $—CH_2—CH_2—$ | (Ia) |
| | $—CH{=}CH—$ | (Ib) |
| | $—CHOH—CH_2—$ | (Ic) |
| | $—CO—CH_2—$ | (Id) |
| $R^1$ | $—CH_2—O—R'$ | |
| | 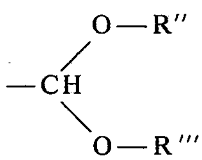 | |

wobei R′ für eine $C_1—C_4$-Alkylgruppe oder Arylmethylgruppe steht und R″ und R‴ $C_1—C_4$-Alkylgruppen bedeuten, die auch zu einem fünf- oder sechsgliedrigen cyclischen Acetal verbunden sein können

$R^2$ $C_1—C_4$-Alkyl
$R^2$ H; Schutzgruppe.

Ferner betrifft die Erfindung die Herstellung der 2-Hydroxymethyl- bzw. 2-Formylchromanderivate II a und II b

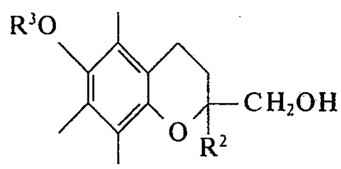

(IIa)

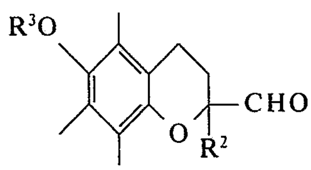

(IIb)

Die Verbindungen II a und II b dienen als wichtige Bausteine für die Synthese des $\alpha$-Tocopherols III

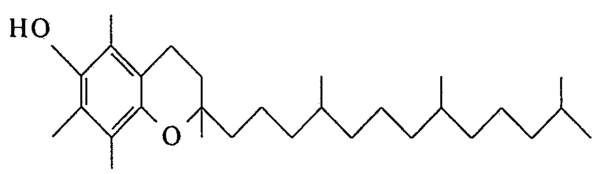

(III)

bzw. seiner 2-Alkylhomologen, indem man z. B.

- die Methylolgruppe von II a in eine Halogenmethylgruppe überführt und die Seitenkette des Tocopherols mittels einer Grignard-Reaktion an das Halogenmethyl-Chromanderivat kuppelt, oder indem man
- II b mit einem 3,7,11-Trimethyldodecylphosphoniumsalz zu III reagieren läßt (H. Mayer et al., Helv. Chim. Acta, Band 46 (1963), S. 650ff.).

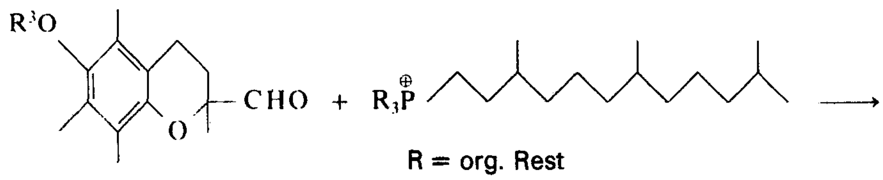

(III)

R = org. Rest

Da das Formylchroman II b und damit das hieraus durch Reduktion erhältliche Hydroxymethylchroman II a bisher nur äußerst schwierig erhältlich sind (vgl. H. Mayer et al., S. 652), lag der Erfindung die Aufgabe zugrunde, diese Verbindungen leichter zugänglich zu machen.

Demgemäß wurde gefunden, daß man die 2-Hydroxymethylchromane bzw. die 2-Formylchromane der allgemeinen Formeln II a und II b

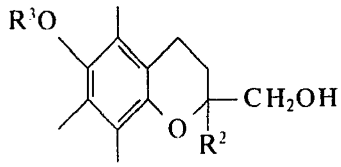

(IIa)

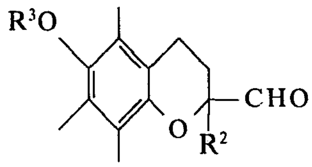

(IIb)

in denen

$R^2$ eine $C_1$ — $C_4$-Alkylgruppe,
$R^3$ Wasserstoff oder eine Schutzgruppe

bedeuten, erhält, wenn man

a) Acetyl-trimethylhydrochinon IV

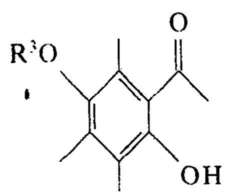

(IV)

in Gegenwart einer Base mit einem Keton der allgemeinen Formel V

$R^2$ — CO — $R^1$ (V)

in der

$R^1$ die Reste

— $CH_2$ — O — R' und

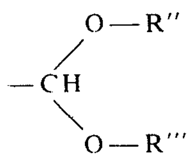

bedeuten, wobei R' für eine $C_1$ — $C_4$-Alkylgruppe oder Arylmethylgruppe steht und R'' und R''' $C_1$ — $C_4$-Alkylgruppen bedeuten, die auch zu einem fünf- oder sechsgliedrigen cyclischen Acetal verbunden sein können

zu den Chromanderivaten Id

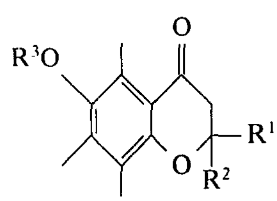

(Id)

umsetzt,

b) I d unter dehydratisierenden Bedingungen zu I a hydriert

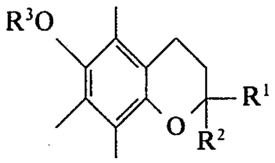

(I a)

und

c) die Reste $R^1$ in den Verbindungen I a durch Hydrolyse oder Hydrogenolyse in die Hydroxmethylgruppe bzw. in die Formylgruppe überführt.

Die Ausgangsverbindung IV, das Acetyl-trimethylhydrochinon, ist bekannt und z. B. vorteilhaft durch Friedel-Crafts-Synthese aus Trimethylhydrochinon und Acetylchlorid erhältlich.

Die Ketone V sind ebenfalls bekannt oder in bekannter Weise zugänglich. Die Alkyl-alkoxymethylketone V a

$$R^2—CO—CH_2—O—R' \qquad (V\,a)$$

können beispielsweise durch Veretherung von Alkan-2-on-1-olen hergestellt werden. Da die Alkoxygruppen (— O — R') im Laufe der meisten weiteren Synthesen, darunter vor allem der erfindungsgemäßen Herstellung der Verbindungen II a und II b, ohnehin wieder hydrolytisch oder hydrogenolytisch abgespalten wird, bevorzugt man als Reste R' naturgemäß leicht abspaltbare Gruppen wie die Methylgruppe, die Benzylgruppe und die tert.-Butylgruppe.

Die Alkyl-dialkoxymethylketone V b

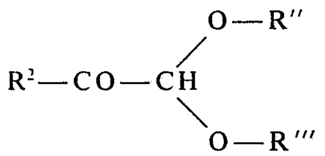

(V b)

sind z. B. nach den Verfahren der DE-OS 12 52 193 und der DE-AS 22 47 345 erhältlich. Als Reste R' kommen vor allem die Methylgruppe und die Benzylgruppe in Betracht. Bevorzugte Reste R'' und R''' sind die Methylgruppe sowie der 1,2-Ethylen-, 1,2-Propylen-, 1,3-Propylen- und besonders der 2,2-Dimetyhl-1,3-propylenrest.

Bevorzugte Reste $R^2$ in den Verbindungen V und damit in den Verbindungen I sind die Ethyl- und vor allem die Methylgruppe.

Es kann als überraschend bezeichnet werden, daß die unter Ringschluß verlaufende Aldolkondensation von IV mit V a und besonders V b glatt gelingt. Die Umsetzung von IV mit Carbonylverbindungen zu Chromanderivaten war zwar für den Fall solcher Carbonylverbindungen, die neben der der Carbonylgruppe keine weiteren reaktionsfähigen Gruppen enthalten, welche die Kondensationsreaktion stören könnten, aus der DE-OS 26 11 910 bekannt, nicht aber mit bifunktionellen Verbindungen wie den Alkoxyketonen V a und den Glyoxalderivaten V b.

Die Umsetzung von IV mit V wird in Gegenwart einer Base vorgenommen. Als Basen eignen sich anorganische Basen wie NaOH, KOH, Na-Methylat und K-Methylat in Mengen von 1 bis 2 mol pro Mol IV sowie besonders organische Amine in Mengen von 1 bis 5 mol pro Mol IV. Besonders empfehlen sich hierbei secundäre Amine wie Pyrrolidin, Piperidin, Morpholin und Dimethylamin.

Man kann die Reaktion ohne Lösungsmittel vornehmen, bevorzugt jedoch das Arbeiten in inerten organischen Lösungsmitteln wie Toluol, Benzol, Tetrahydrofuran, Tetrachlorkohlenstoff und Xylol. Die Menge des Lösungsmittels beträgt hierbei etwa 2 bis 5 l pro kg IV. Die Reaktionstemperaturen liegen zweckmäßigerweise zwischen 50 und 150°C. Es empfiehlt sich, das bei der Reaktion freiwerdende Wasser laufend aus dem Reaktionsgemisch abzudestillieren, z. B. durch azeotrope Destillation mit dem Lösungsmittel. Weiterhin ist es zweckmäßig, die Umsetzung unter Inertgasatmosphäre vorzunehmen, da das Acetyl-trimethylhydrochinon oxidationsempfindlich ist.

Das Mengenverhältnis von IV zu V liegt vorzugsweise zwischen 1 : 1 bis 0,5 : 1.

Eine besondere Ausführungsform des Verfahrensschrittes (a) besteht darin, als Base ein optisch aktives Amin zu verwenden, da hierdurch die Bildung eines der beiden Enantiomeren von I d, dessen 2-C-Atom ein Asymmetriezentrum ist, begünstigt wird, denn hierdurch wird die Synthese von optisch reinem $\alpha$-Tocopherol erleichtert.

Im Verfahrensschritt (b) hydriert man die Chromanone I d unter dehydratisierenden Bedingungen in an sich bekannter Weise zu den Chromanen I a, z. B. mit Metallhydriden wie Diisobutylaluminiumhydrid, $LiAlH_4$ oder mit Wasserstoff bei 50 bis 300 bar und 50 bis 250°C in Gegenwart eines Edelmetallkatalysators wie Pd oder Pt oder von Raney-Nickel.

Man kann die Überführung von I d in I a auch stufenweise vornehmen, indem man I d zunächst mit einem Metallhydrid wie $NaBH_4$ oder mit Wasserstoff in Gegenwart von einem oben genannten Hydrier-Katalysatoren zum 4-Hydroxychroman (I c) hydriert, dieses sodann in an sich bekannter Weise, z. B. mittels Behandlung mit Säure oder durch thermische Dehydratisierung zum Chrom-3-en (I b) dehydratisiert und letzteres in ebenfalls an sich bekannter Weise in Gegenwart eines inerten Lösungsmittels katalytisch mit Wasserstoff zu I a hydriert.

Die stufenweise Reaktion von I d zu I a nimmt folgenden Verlauf:

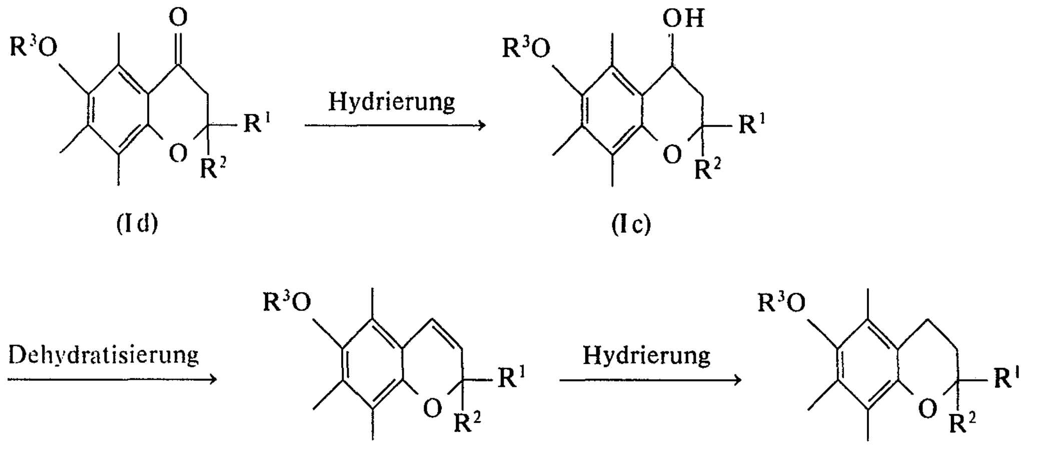


(I d) (I c)

(I b) (I a)

Im Verfahrenschritt (c) werden die Verbindungen I a sodann durch Abspaltung des Etherrestes bzw. der Acetalgruppen in ebenfalls an sich bekannter Weise zu den 2-Hydroxymethyl- bzw. 2-Formylchromanen II a und II b umgesetzt, indem man sie beispielsweise in wäßriger Lösung mit Mineralsäuren behandelt.

Man kann bei der von IV ausgehenden Synthese die freie 6-Hydroxy-Verbindung oder das mittels des Restes $R^3$ geschützte Acetyltrimethylhydrochinon einsetzen. Geht man von der ungeschützten Verbindung aus ($R^3 = H$), so kann der Rest $R^3$ in an sich bekannter Weise auf einer beliebigen Stufe der Synthese eingeführt und gewünschtenfalls auch wieder hydrolytisch oder hydrogenolytisch abgespalten werden. Als Schutzgruppen $R^3$ kommen, wie allgemein bekannt ist, z. B. $C_1$ — $C_4$-Alkylgruppen wie die Methylgruppe, Arylmethylgruppen wie die Benzylgruppe, Tri-($C_1$ — $C_4$-alkyl)-silylgruppen wie die Trimethylsilylgruppe, $C_1$ — $C_4$-Acylgruppen wie die Acetylgruppe, die Tetrahydropyran-2-yl-gruppe und die 4-Methyl-5,6-dihydro-2-H-pyran-2-yl-gruppe in Betracht.

Bevorzugte Schutzgruppen sind die Acetyl-, Benzyl- und tert.-Butylgruppe.

Die Verbindungen I (I a—I d) sind wertvolle Zwischenprodukte für die Synthese des $\alpha$-Tocopherols (Vitamin E) und seiner Derivate. Ein wichtiger Synthesenweg verläuft über die Verbindungen II a oder II b, jedoch ist es auch möglich, die Seitenkette des Tocopherols in die Verbindungen I b, I c oder I d einzuführen und die zur Herstellung der Verbindungen mit Chromanstruktur erforderlichen Operationen anschließend vorzunehmen.

## Beispiele

Alle Operationen wurden unter Argonatmosphäre vorgenommen.

### Beispiel 1

Herstellung von 2-Ethyl-2-(5′,5′-dimethyl-1′,3′-dioxan-2′-yl)-6-hydroxy-5,7,8-trimethylchroman-4-on

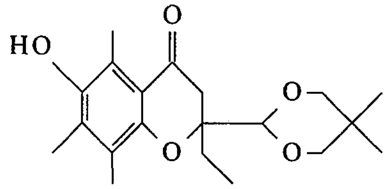

3,00 g (15,6 mmol) Acetyl-trimethylhydrochinon und 3,10 g (6,18 mmol) 5,5-Dimethyl-2-(propion-1′-yl)-1,3-dioxan wurden zusammen mit 2,50 g (30 mmol) Pyrrolidin und 45 ml Toluol 3 h lang zum Sieden erhitzt, wobei das entstehende Wasser laufend als Wasser/Toluol-Azeotrop entfernt wurde.

Das Reaktionsgemisch wurde sodann auf Eiswasser gegossen und mit verdünnter Salzsäure auf pH 3 gestellt, wonach das Verfahrensprodukt in üblicher Weise mit Methylenchlorid extrahiert und aus der Extraktphase isoliert wurde.
Sdp. 250°C (Kugelrohr); weiße Kristalle, Fp. 141°C, Ausbeute 76%.

## Beispiel 2

Herstellung von 2-(1',1'-Dimethoxymethyl)-6-hydroxy-2,5,7,8-tetramethyl-chroman-4-on

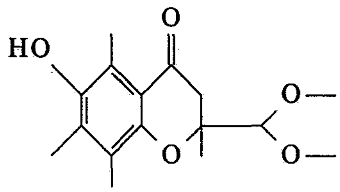

Analog Beispiel 1 wurden 15,0 g (77 mmol) Acetyl-trimethylhydrochinon und 10,5 g (89 mmol) 1,1-Dimethoxy-propan-2-on zusammen mit 9,30 g (131 mmol) Pyrrolidin in toluolischer Lösung zu dem obengenannten Verfahrensprodukt umgesetzt.
Sdp. 165—170°C/6 · $10^{-5}$ mbar (Kugelrohr); Ausbeute 70%.

## Beispiel 3

Herstellung von 6-Hydroxy-2-methoxymethyl-2,5,7,8-tetramethyl-chroman-4on

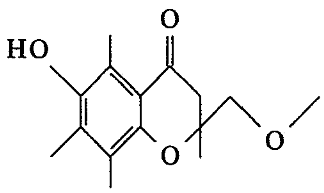

Analog Beispiel 1 wurden 3,00 g (15,6 mmol) Acetyl-trimethylhydrochinon und 1,58 g (18 mmol) Methoxyaceton zusammen mit 2,50 g (30 mmol) Pyrrolidin in toluolischer Lösung zu dem obengenannten Verfahrensprodukt umgesetzt, welches in öliger Form anfiel.
Sdp. 250—270°C (Kugelrohr); Ausbeute 37%.

## Beispiel 4

Herstellung von 2-tert.-Butoxymethyl-6-hydroxy-2,5,7,8-tetramethyl-chroman-4-on

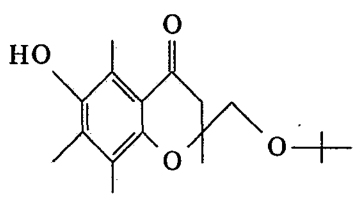

Analog Beispiel 1 wurden 1,00 g (5,2 mmol) und 1,01 g (7,8 mmol) tert.-Butoxyaceton zusammen mit 0,92 g (13 mmol) Pyrrolidin in toluolischer Lösung zu den obengenannten Verfahrensprodukten umgesetzt. Es fiel ein öliges Rohprodukt an (Kugelrohr); Ausbeute 19%; IR: 3600, 1720 $cm^{-1}$.

## Beispiel 5

Herstellung von optisch angereichertem
6-Acetoxy-2-(1',1'-dimethoxymethyl)-2,5,7,8-tetramethyl-chroman-4-on

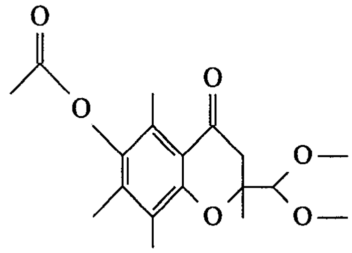

1,00 g (5,2 mmol) Acetyl-trimethylhydrochinon und 0,71 g (6 mmol) 1,1-Dimethoxypropanon wurden zusammen mit 1,65 g (10 mmol) (L)-Phenylanalin und 20 ml Acetonitril sowie 2 g 4-Å-Molekularsieb (zur Bindung des Reaktionswassers) 100 h unter Rückflußkühlung gerührt.

Die bei der Aufarbeitung des Reaktionsgemisches erhältliche rohe 6-Hydroxyverbindung wurde mit 1,5 g Acetanhydrid und in bekannter Weise mit einem Basengemisch aus 5 ml Pyridin und 0,5 g 4-(N,N-Dimethylamino)-pyridin acetyliert. Das so erhaltene Gemisch wurde mit verdünnter Salzsäure versetzt, wonach das Verfahrensprodukt mit Ether extrahiert und wie üblich aus der Extraktphase gewonnen wurde. Nach Kugelrohrdestillation bei 250°C/4 · $10^{-5}$ mbar wurden 0,62 g teilkristallines Öl (54%d. Th.) erhalten. Dieses Produkt wurde durch Chromatographie an 120 g Kieselgel mit Essigester/Hexan (50/50 gereinigt; weiße Kristalle, Fp. 100—101°C.

Der Drehwert von $[\alpha]_D^{25} = 0{,}51°$ (c = 2,12/$CH_2Cl_2$) läßt auf eine optische Anreicherung eines der Chroman-Enantiomeren, verursacht durch optische Induktion der chiralen Base, schließen.

## Beispiel 6

Herstellung von 2-(1',1'-Dimethoxymethyl)-4,6-dihydroxy-2,5,7,8-tetramethylchroman

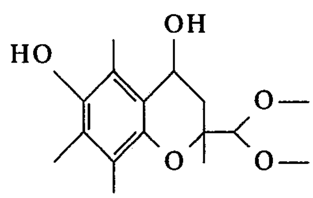

9,4 g (32 mmol) des Verfahrensproduktes von Beispiel 2 wurden bei 20—60°C in 100 ml Methanol mit 2,43 g (64 mmol) $NaBH_4$ umgesetzt. Nach 6 h wurden weitere 2,43 g $NaBH_4$ zugesetzt, wonach das Reaktionsgemisch 16 h bei Raumtemperatur gerührt wurde. Anschließend wurde es mit 100 ml 3n-NaOH und danach zur Neutralisation mit verdünnter Salzsäure versetzt und nach einer Extraktion mit Ether unterworfen, wobei ein braunes Öl als Rohprodukt in 95%iger Ausbeute anfiel.
IR: 3600 (breit) $cm^{-1}$.

## Beispiel 7

Herstellung von 2-(1',1'-Dimethoxymethyl)-6-hydroxy-2,5,7,8-tetramethyl-chrom-3-en

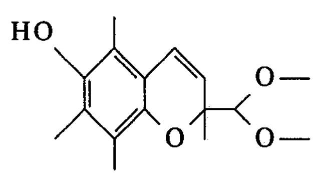

8,9 g (30 mmol) des nach Beispiel 6 erhaltenen 4-Hydroxychromans wurden im Kugelrohr zweimal bei 150—200°C/$10^{-3}$ mbar destilliert. Dabei wurde ein gelbes, teilweise kristallisierendes Öl erhalten (53% Ausbeute), das durch Chromatographie an $Al_2O_3$ mit einem Gemisch aus 2 Teilen Ether und 1 Teil Hexan gereinigt wurde. Fp. 105—107°C.

## Beispiel 8

Herstellung von 2-(1',1'-Dimethoxymethyl)-6-hydroxy-2,5,7,8-tetramethyl-chroman

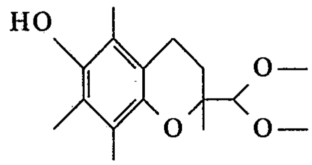

1,74 g (0,63 mmol) des nach Beispiel 7 erhaltenen Chromens wurden in 30 ml Methanol bei Raumtemperatur und Normaldruck im Laufe von 16 h mittels 0,15 g eines Pd/Aktivkohleträgerkatalysators, dessen Pd-Gehalt 10 Gew.-% betrug, hydriert. Die übliche Aufarbeitung lieferte die obengenannte Verbindung in 97%iger Ausbeute. Weiße Kristalle, Fp. 102—106°C.

## Beispiel 9

Herstellung von 6-Benzyloxy-2-(1',1'-dimethoxymethyl)-2,5,7,8-tetramethyl-chroman

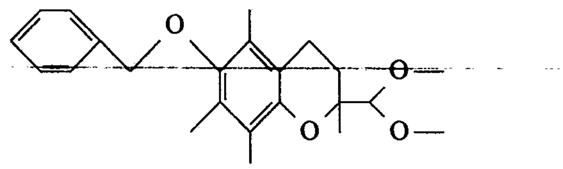

1,47 g (5,25 mmol) des nach Beispiel 8 erhaltenen Chromans wurden mit 3,2 g Benzylbromid, 1,5 g $Na_2CO_3$ und 7 ml Dimethylformamid 13 h auf 100°C erhitzt und danach mit Wasser versetzt. Dieses Gemisch wurde mit Heptan extrahiert, wonach das aus der Extraktphase gewonnene Rohprodukt mit einer Mischung aus 1 Volumenteil Ether und 2 Volumenteilen Heptan an 50 g Kieselgel chromatographiert wurde. Hierbei wurde folgende Fraktionen erhalten: F 1 = 1,28 g; F 2 (Mischfraktion) 0,13 g und F 3 (Ausgangsmaterial 0,23 g.

Die Fraktionen F 1 und F 2 wurden an 100 g Kieselgel rechromatographiert, wobei das obengenannte Verfahrensprodukt in 64%iger Ausbeute anfiel. Weiße Kristalle, Fp. 46—48°C.

## Beispiel 10

Herstellung von 6-Benzyloxy-2-formyl-2,5,7,8-tetramethyl-chroman

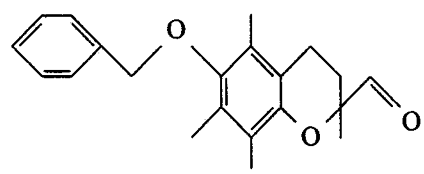

Ein Gemisch aus 0,16 g (0,43 mmol) der nach Beispiel 9 erhaltenen Verbindung, 4 ml Ether und 2 ml konzentrierter Salzsäure wurden 2 h auf 40°C erwärmt, danach mit Ether verdünnt und mit wäßriger $NaHCO_3$-Lösung gewaschen. Aus der verbleibenden organischen Phase wurde die obengenannte Verbindung wie üblich isoliert; Ausbeute 48%.

## Beispiel 11

Herstellung von 2-(1',1'-Dimethoxymethyl)-6-hydroxy-2,5,7,8-tetramethyl-chroman
(Formel s. Beispiel 8)

0,50 g (1,7 mmol) des Chromanons gemäß Beispiel 2 wurden im Laufe von 12 h bei 250 bar und 180°C in Gegenwart von 1 g Raney-Nickel hydriert. Die chromatographische Reinigung des Rohproduktes lieferte das oben genannte Chroman, dessen Eigenschaften mit denen des Verfahrensproduktes von Beispiel 8 übereinstimmten.

Beispiel 12

Herstellung von 6-Benzyloxy-2-tert.-butoxymethyl-2,5,7,8-tetramethyl-chroman-4-on

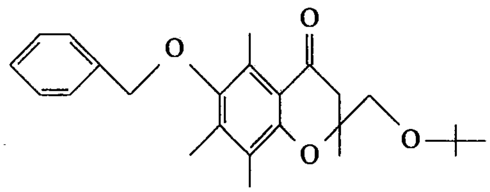

3,5 g des rohen Verfahrensproduktes von Beispiel 4 wurden analog Beispiel 9 mit 7 g Benzylbromid, 3 g $Na_2CO_3$ und 15 ml Dimethylformamid in das oben genannte Chromanon überführt. Ausbeute (nach dreifacher chromatographischer Reinigung): 20%; Fp. 51—62°C.

Beispiel 13

Herstellung von 6-Benzyloxy-2-hydroxymethyl-2,5,7,8-tetramethylchroman

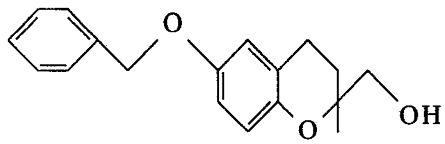

0,90 g (2,20 mmol) des nach Beispiel 12 erhaltenen Chromanons wurden analog der Beispiele 6 bis 8 in das 2-tert.-Butoxymethyl-chromanderivat überführt, wonach das so erhaltene Rohprodukt 2 h lang bei 0°C mit Trifluoressigsäure gerührt wurde. Anschließend wurde das Gemisch auf 50 ml Eiswasser gegossen und hieraus mit 50 ml Methylenchlorid extrahiert. Die chromatographische Reinigung lieferte die oben genannte Verbindung in Form weißer Kristalle, Fp. 65—68°C, Ausbeute 30%.

**Patentansprüche**

1. Chromanderivate der allgemeinen Formel I

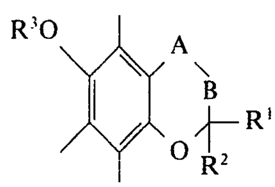

(I)

in welcher die Substituenten folgende Bedeutung haben:

A—B $-CH_2-CH_2-$ (Ia)

$-CH{=}CH-$ (Ib)

$-CHOH-CH_2-$ (Ic)

$-CO-CH_2-$ (Id)

$R^1$ $-CH_2-O-R'$

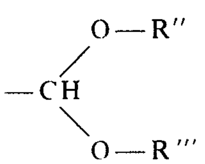

wobei R' für eine $C_1-C_4$-Alkylgruppe oder Arylmethylgruppe steht und R'' und R''' $C_1-C_4$-Alkylgruppen bedeuten, die auch zu einem fünf- oder sechsgliedrigen cyclischen Acetal verbunden sein können

$R^2$ $C_1-C_4$-Alkyl
$R^3$ H; Schutzgruppe.

2. Chromanderivate der allgemeinen Formel I'

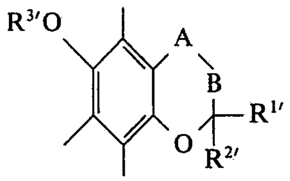

(I')

in denen die Substituenten $R^{1'}$–$R^{3'}$ folgende Bedeutung haben:

$R^{1'}$

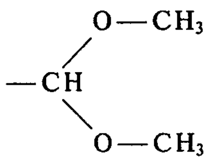

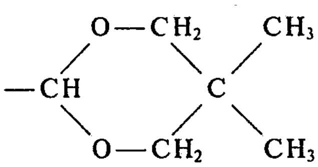

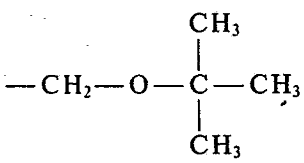

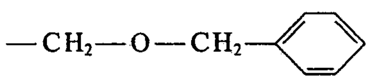

$R^{2'}$ Methyl, Ethyl

$R^{3'}$ H; Acetyl, Benzyl,

und A–B die dort angegebenen Bedeutungen haben.

3. Verfahren zur Herstellung von 2-Hydroxymethylchromanen und 2-Formylchromanen der allgemeinen Formeln II a und II b

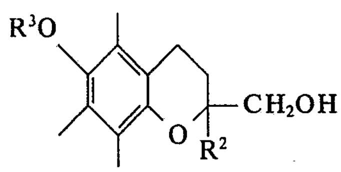

(IIa)

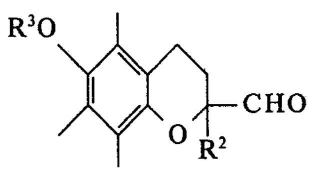

(IIb)

in denen

$R^2$ eine $C_1-C_4$-Alkylgruppe und
$R^3$ Wasserstoff oder eine Schutzgruppe

bedeuten, dadurch gekennzeichnet, daß man

a) Acetyltrimethylhydrochinon IV

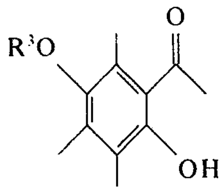

(IV)

in Gegenwart einer Base mit einem Keton der allgemeinen Formel V

$R^2—CO—R^1$ (V)

in der

$R^1$ die Reste

$—CH_2—O—R'$ und

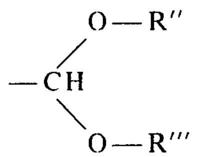

bedeuten, wobei R' für eine $C_1—C_4$-Alkylgruppe oder Arylmethylgruppe steht und R'' und R''' $C_1—C_4$-Alkylgruppen bedeuten, die auch zu einem fünf- oder sechsgliedrigen cyclischen Acetal verbunden sein können

zu den Chromanderivaten Id

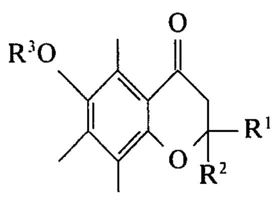

(Id)

umsetzt,

b) Id unter dehydratisierenden Bedingungen zu Ia hydriert

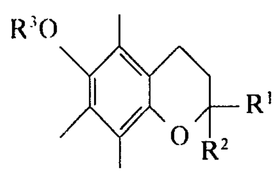

(Ia)

und

c) die Reste $R^1$ in den Verbindungen Ia durch Hydrogenolyse oder Hydrolyse in die Hydroxmethylgruppe bzw. in die Formylgruppe überführt.

4. Verfahren nach Anspruch 3, dadurch gekennzeichnet, daß man im Verfahrensschritt (a) eine optisch aktive organische Base verwendet.

**Claims**

1. A chroman derivative of the general formula I

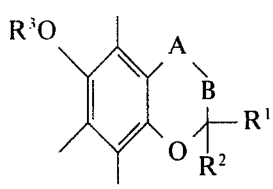

(I)

where

A—B is $—CH_2—CH_2—$ (Ia)

$—CH{=}CH—$ (Ib)

$—CHOH—CH_2—$ (Ic)

or

$—CO—CH_2—$ (Id)

$R^1$ is $—CH_2—O—R'$

or

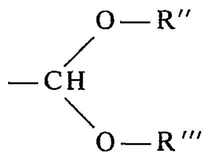

where R′ is $C_1—C_4$-alkyl or arylmethyl and R″ and R‴ are each $C_1—C_4$-alkyl, which can also be bonded to form a five-membered or six-membered cyclic acetal, $R^2$ is $C_1—C_4$-alkyl and $R^3$ is H or a protective group.

2. A chroman derivative of the general formula I′

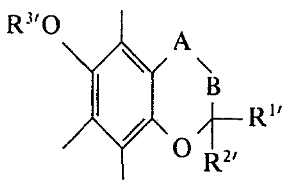

(I′)

where $R^{1'}$ is

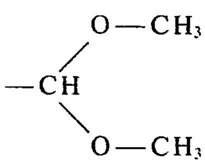

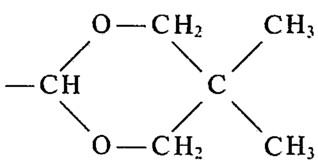

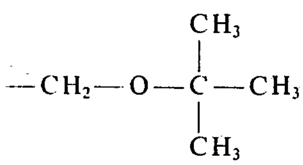

or

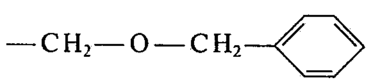

$R^{2'}$ is methyl or ethyl, $R^{3'}$ is H, acetyl or benzyl, and A—B has the meanings indicated above.

3. A process for the preparation of a 2-hydroxymethylchroman or a 2-formylchroman of the general formula II a or II b

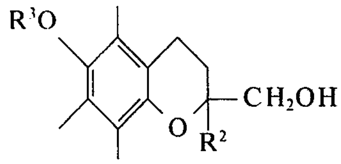

(IIa)

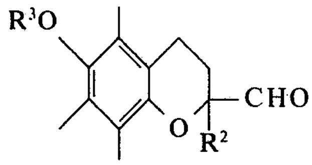

(IIb)

where $R^2$ is $C_1$—$C_4$-alkyl and $R^3$ is hydrogen or a protective group, wherein

a) acetyl-trimethylhydroquinone IV

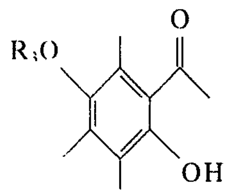

(IV)

is reacted with a ketone of the general formula V

$R^2$—CO—$R^1$ (V)

where $R^1$ is —$CH_2$—O—R′ or

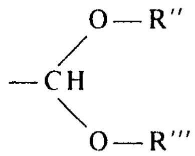

where R′ is $C_1$—$C_4$-alkyl or arylmethyl and R″ and R‴ are each $C_1$—$C_4$-alkyl, which can also be bonded to form a five-membered or six-membered cyclic acetal, in the presence of a base to give a chroman derivative Id

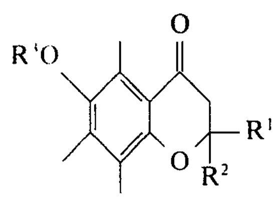

(Id)

b) Id is hydrogenated under dehydrating conditions to give Ia

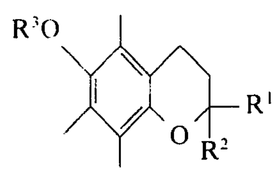

(Ia)

and

c) $R^1$ in Ia is converted into hydroxymethyl by hydrolysis or into formyl by hydrogenolysis.

4. A process as claimed in claim 3, wherein an optically active organic base is used in step (a).

**Revendications**

1. Dérivés de chromannes de la formule générale I

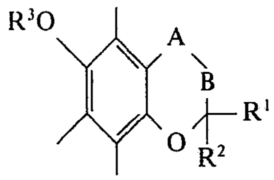

(I)

dans laquelle les substituants possèdent les significations ci-après:

| | | |
|---|---|---|
| A—B | $-CH_2-CH_2-$ | (Ia) |
| | $-CH=CH-$ | (Ib) |
| | $-CHOH-CH_2-$ | (Ic) |
| | $-CO-CH_2-$ | (Id) |
| $R^1$ | $-CH_2-O-R'$ | |

ou 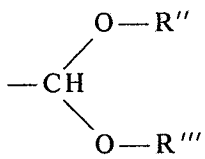

R′ désignant un radical alkyle en $C_1$ à $C_4$ ou un groupe aryl-méthyle et R″ et R‴ des radicaux alkyle en $C_1$ à $C_4$, pouvant éventuellement former ensemble un acétal cyclique penta- ou hexagonal;

$R^2$ alkyle en $C_1$ à $C_4$ et

$R^3$ H ou groupe protecteur.

2. Dérivés de chromannes de la formule générale I′

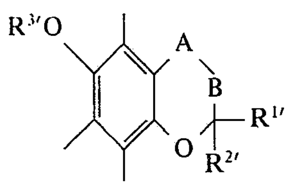

(I′)

dans laquelle les substituants $R^{1'}$ à $R^{3'}$ possèdent les significations ci-après:

$R^{1'}$ 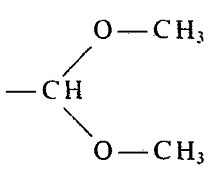

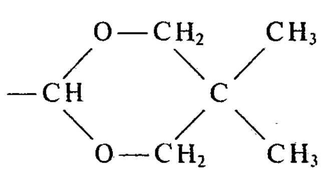

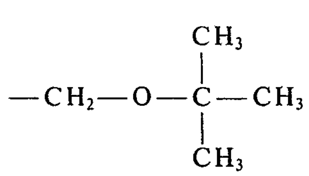

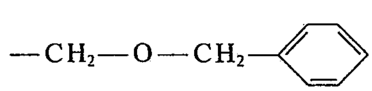

$R^{2\prime}$ méthyle ou éthyle;
$R^{3\prime}$ H, acétyle ou benzyle,

A—B possédant les significations données dans la revendication 1.

3. Procédé de préparation de 2-hydroxyméthyl-chromannes et de 2-formyl-chromannes des formules générales II a et II b

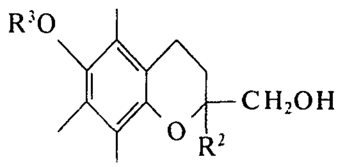

(IIa)

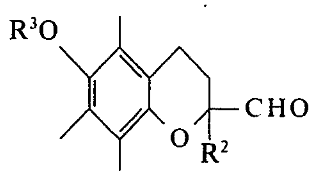

(IIb)

dans lesquelles $R^2$ représente un radical alkyle en $C_1$ à $C_4$ et $R^3$ un atome d'hydrogène ou un groupe protecteur, caractérisé en ce que:

a) on fait réagir une acétyl-triméthyl-hydroquinone IV

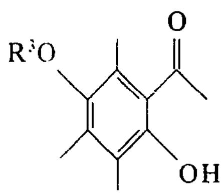

(IV)

en présence d'une base avec une cétone de la formule générale V

$R^2—CO—R^1$ (V)

dans laquelle $R^1$ désigne un reste

$—CH_2—O—R'$

ou un reste

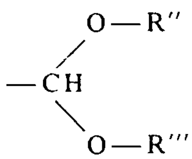

R' désignant un radical alkyle en $C_1$ à $C_4$ ou un groupe aryl-méthyle et R'' et R''' des radicaux alkyle en $C_1$ à $C_4$, pouvant éventuellement former ensemble un acétal cyclique penta- ou hexagonal;

b) on soumet les dérivés du chromanne obtenus, de la formule I d

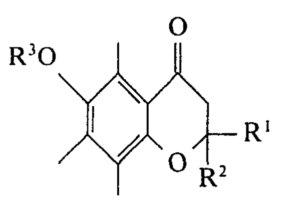

(Id)

à une hydrogénation dans des conditions déshydratantes pour l'obtention des dérivés de la formule I a

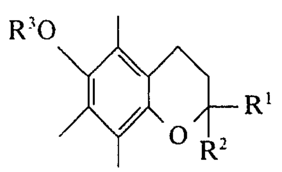

(I a)

et

c) on transforme le substituant $R^1$ des composés de la formule I a par hydrogénolyse ou hydrolyse en un radical hydroxy-méthyle ou un groupe formyle.

4. Procédé suivant la revendication 3, caractérisé en ce que, dans le stade opératoire (a), on emploie une base organique optiquement active.